# EUROPÄISCHE PATENTSCHRIFT

(11) **EP 1 168 972 B1**
(45) Veröffentlichungstag und Bekanntmachung des Hinweises auf die Patenterteilung: **14.03.2007**
(21) Anmeldenummer: 00917032.5
(22) Anmeldetag: 31.03.2000
(51) Int. Cl.: A61B 18/14

(54) **CHIRURGISCHES INSTRUMENT**
SURGICAL INSTRUMENT
INSTRUMENT CHIRURGICAL

(30) Priorität: 01.04.1999 DE 19915062
(43) Veröffentlichungstag der Anmeldung: 09.01.2002
(73) Patentinhaber: Erbe Elektromedizin GmbH, 72072 Tübingen (DE)
(72) Erfinder: FARIN, Günter, D-72070 Tübingen (DE)
(74) Vertreter: Bohnenberger, Johannes
(86) Internationale Anmeldenummer: PCT/EP2000/002907
(87) Internationale Veröffentlichungsnummer: WO 2000/059391

(56) Entgegenhaltungen:
- EP-A- 0 400 288
- EP-A- 0 795 301
- WO-A-97/17033
- US-A- 2 068 721
- US-A- 5 718 703

## Beschreibung

Die Erfindung betrifft ein chirurgisches Instrument, das eine Vielzahl von Funktionen aufweist.

Aus der US-PS 40 43 342 ist ein Schneidinstrument für die Hochfrequenz-Chirurgie bekannt, bei welchem eine als Schneidelektrode dienende dünne metallische Nadel aus einer als Neutralelektrode dienenden Metallhülse, gegen welche sie isoliert ist, in axialer oder radialer Richtung herausragt bzw. herausgeschoben werden kann. Ein derartiges Instrument ist beispielsweise für feine Schnitte geeignet. Je dünner die metallische Nadel ist, desto weniger Hochfrequenzsstrom ist zum Schneiden erforderlich und desto geringer werden die Schnittränder thermisch geschädigt. Eine dünne metallische Nadel erhöht jedoch das Risiko eines Verbiegens oder Abbrechens, so daß leicht unbeabsichtigt Gewebe verletzt werden kann. Ein stumpfes Präparieren ist mit der Anordnung nicht möglich, so daß bei Bedarf ein anderes Instrument verwendet werden muß. Ein derartiger Instrumentenwechsel ist zeit- und arbeitsaufwendig, was die Risiken für den Patienten erhöht.

Aus der US 49 31 047 ist ein chirurgisches Instrument bekannt, bei welchem zur Vermeidung eines inter-operativen Instrumentenwechsels über die selbe Applikatorspitze eines multifunktionalen Instrumentes Ultraschall oder ein Hochfrequenzstrom gleichzeitig oder abwechselnd appliziert werden können. Ein stumpfes Präparieren ist auch mit diesem Instrument nicht möglich.

Zum stumpfen Präparieren ist eine Vielzahl von Instrumenten bekannt, jedoch müssen diese während der Operation gewechselt werden, wenn man abwechselnd Gewebe zum Beispiel mittels eines Haken aufnehmen, aufspreizen oder ergreifen will.

Aus der US-A-2 068 721 ist ein Instrument bekannt, das Zangenelemente zum Greifen von Gewebe und einen Schneidgrad aufweist, der das Gewebe zwischen den Zangenelementen durchtrennen kann. Die Zangenelemente sind als Elektroden ausgebildet, so dass das erfasste Gewebe vor dem Durchtrennen koaguliert werden kann. Ein stumpfes Präparieren ist mit diesem Instrument nicht möglich.

Aus der US-A-5 718 703 ist ein chirurgisches Instrument nach dem Oberbegriff des Patentanspruches 1 bekannt. Die dort vorgesehenen Zangenelemente werden alle gleichzeitig bewegt, so dass die das Gewebe erfassenden Paare von Zangenelementen während des Zupackens immer näher zusammenrücken. Dadurch wird ein Bearbeiten des Gewebes zwischen den Zangenelementen zumindest erschwert. Darüber hinaus können bei dem bekannten Instrument die zwei mit Haken versehenen Zangenelemente nur in gespreiztem Zustand zum stumpfen Präparieren verwendet werden, was diesen Vorgang erschwert.

Der Erfindung liegt die Aufgabe zu Grunde, ein Instrument nach dem Oberbegriff des Patentanspruches 1 dahin gehend weiter zu bilden, dass sowohl der Koagulationsvorgang, als auch der Präparationsvorgang erleichtert werden.

Diese Aufgabe wird durch das chirurgische Instrument nach dem Patentanspruch 1 gelöst.

Vorzugsweise sind an den distalen Abschnitten Elektrodeneinrichtungen vorgesehen bzw. sind die distalen Abschnitte als Elektrodeneinrichtungen ausgebildet, welche mit steuerbaren HF-Generatoren zum Zuführen eines Hochfrequenzstroms insbesondere zum Koagulieren von Gewebeabschnitten verbunden sind, die von den Zangenelementen erfaßt werden oder an welche die Zangenelemente angelegt werden. Vorzugsweise sind hierbei die proximalen Abschnitte im wesentlichen ringsum isoliert, so daß unbeabsichtigte Verletzungen vermieden werden können.

Bei einer besonders bevorzugten Ausführungsform der Erfindung ist eine Schneideinrichtung vorgesehen, die gegenüber den Zangenelementen zum Schneiden von Gewebe bewegbar ausgebildet ist. Es kann also mit dem Instrument einerseits stumpf präpariert, andererseits das so zum Beispiel auf den Haken aufgenommene Gewebe auch geschnitten werden. Vorzugsweise umfaßt hierfür die Schneideinrichtung eine Elektrode bzw. ist als Elektrode ausgebildet und mit einem steuerbare HF-Generator zum Durchtrennen oder Schneiden von Gewebe mittels eines hochfrequenten elektrischen Stroms verbunden.

Eine solche Schneideinrichtung kann von außen in Richtung auf die distalen Abschnitte zugeführt werden, jedoch ist es von besonderem Vorteil, wenn die Schneideinrichtung zwischen den distalen Abschnitten und/oder den proximalen Abschnitten aufnehmbar ausgestaltet ist, wenn diese zu einem Haken zusammengeführt sind. Dadurch kann eine unbeabsichtigte Verletzung von Gewebe nicht geschehen. Darüber hinaus ist dann, wenn die Schneideinrichtung als dünner Draht ausgeführt ist, gleichzeitig eine Verletzungsgefahr für den Operateur im "Ruhezustand" des Gerätes also bei zusammengelegten distalen Abschnitten nicht mehr gegeben.

Vorzugsweise umfassen die Betätigungseinrichtungen der proximalen Abschnitte einen rohrförmigen Abschnitt, in welchem die proximalen Abschnitte geführt und gehalten sind. Die Bewegungen der proximalen und damit auch der distalen Abschnitte können in an sich bekannter Weise induziert werden. Am proximalen Ende des rohrförmigen Abschnittes sind entsprechende Manipulatoreinrichtungen zum Bewegen der distalen Abschnitte vorgesehen.

Das Instrument ist vorzugsweise derart modular aufgebaut, daß die hygienerelevanten Teile schnell, leicht und sicher desinfiziert und sterilisiert werden können. Dies wird vorzugsweise dadurch erreicht, daß die Effektoren, also im wesentlichen die proximalen Abschnitte inclusive Rohrschaft von den Aktoren trennbar ausgebildet sind. Hierdurch können die Reinigung und die Sterilisation beherrscht werden.

Der rohrförmige Abschnitt kann zusätzlich noch zum Zuführen einer Spüllösung oder eines Schutzgases (zum Koagulieren oder Schneiden) dienen oder aber mit Saugeinrichtungen derart verbunden sein, daß Körperflüssigkeiten oder zuvor eingeführte Spülflüssigkeit abgesaugt werden können.

Die Zuführung von Flüssigkeit ebenso wie von (Schutz-) Gas kann auch gleichzeitig zur Kühlung der Instrumente dienen, wenn ein Hochfrequenzstrom zugeführt wird.

Für die Gestaltung der Form der distalen Abschnitte gibt es je nach Anwendungszweck des erfindungsgemäßen Instrumentes verschiedene Möglichkeiten. Wird das Instrument vorwiegend zum Präparieren angewendet, so könne die distalen Abschnitte optimal für diesen Zweck gestaltet werden. Hierbei wird beispielsweise von einem runden, einstückigen Präparationshaken ausgegangen, der in Längsrichtung derart geteilt wird, daß mehrere gleiche oder auch unterschiedliche Teile, nachfolgend Stifte genannt, entstehen. Wird das Instrument vorwiegend zum Trennen bzw. Schneiden von Gewebestrukturen angewendet, wobei eine prophylaktische Hämostase durch thermische Koagulation durchgeführt werden muß, dann können die Stifte optimal für diesen Zweck geformt werden. Hierfür können beispielsweise zwei Stifte bzw. ein Stiftpaar eine runde zylinderische Form und zwei andere Stifte bzw. ein zweites Stiftpaar die Form einer zylinderischen Schale haben, wobei das zum Zwecke einer prophylaktischen Hämostase thermisch zu koagulierende Gewebe jeweils zwischen einem runden zylinderischen Stift und einer zylinderischen Schale gefaßt wird.

Um die Effektoren, also die distalen Abschnitte und gegebenenfalls die Schneidelektrode(n) dem jeweiligen Anwendungszweck entsprechend koordiniert und sicher in die jeweilige Arbeits- oder Ruhestellung zu führen, sind am proximalen Ende des Rohrschaftes die genannten Aktoren angeordnet. Als manuelle Aktoren eignen sich hierbei Hebel, Tasten, Fingerösen oder Schieber während als automatische Aktoren pneumatische, hydraulische, elektromagnetische und/oder elektromotorische Engergie-/Kraft-Wandler eingesetzt werden. Die Aktivierung der Aktoren erfolgt entweder separat oder in vorprogrammierter Reihenfolge automatisch, beispielsweise über Tasten, Schalter oder auch über verbale Befehle.

Nachfolgend werden Ausführungsformen der Erfindung anhand von Abbilungen näher erläutert. Hierbei zeigen
- Fig.1: eine perspektivische Schemazeichnung eines Endabschnitts des chirurgischen Instrumentes,
- Fig. 2: eine Darstellung zur Erläuterung der Funktionsweise des Instrumentes bei vollständig auseinandergefahrenen distalen Abschnitten,
- Fig. 3: eine Darstellung ähnlich der nach Fig. 2 jedoch mit zwei paarweise zusammengefahrenen distalen Abschnitten,
- Fig. 4: eine perspektivische Darstellung ähnlich der nach Fig. 1 jedoch einer weiteren Ausführungsform der Erfindung,
- Fig. 5: eine Darstellung des Instrumentes nach Fig. 4 in einer Position entsprechend der nach Fig. 3,
- Fig. 6: eine Darstellung einer weiteren Ausführungsform der Erfindung ähnlich der nach Fig. 1-3 jedoch mit einer zusätzlichen Schneidelektrode,
- Fig. 7: einen Schnitt durch das Instrument nach Fig. 1 entlang der Ebene VII-VII und
- Fig. 8 und 9: Schnittdarstellungen ähnlich denen nach Fig. 7 jedoch durch weitere Ausführungsformen der Erfindung.

In der nachfolgenden Beschreibung werden für gleiche und gleichwirkende Teile die selben Bezugsziffern verwendet.

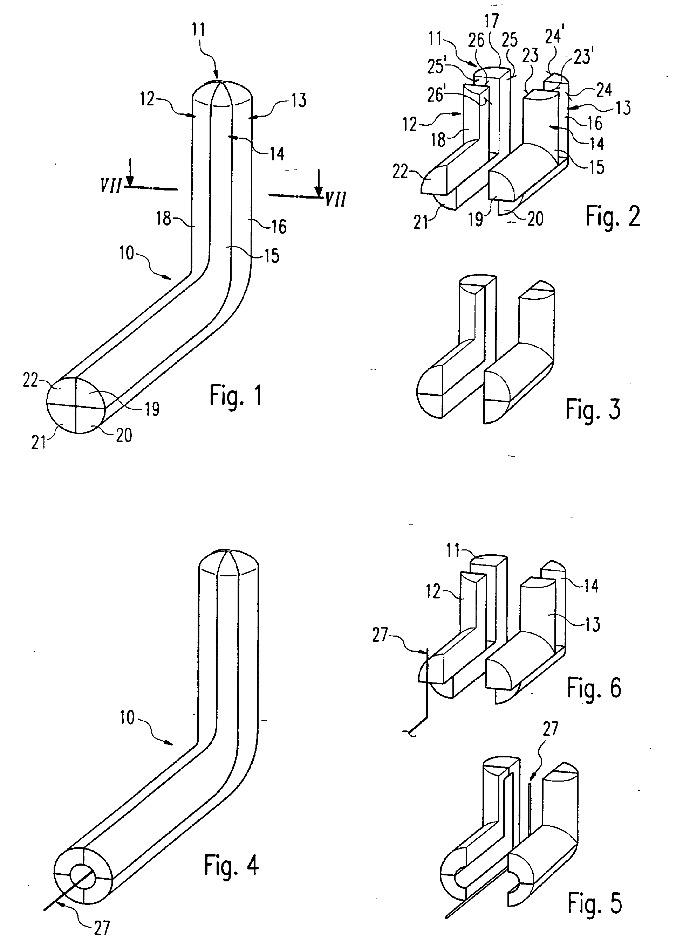

Bei dem in Fig. 1 gezeigten chirurgischen Instrument wird ein Haken 10 aus vier Zangenelementen 11, 12, 13 und 14 gebildet, die jeweils einen distalen Abschnitt 15, 16, 17 und 18 sowie einen proximalen Abschnitt 19, 20, 21 und 22 umfassen. Die proximalen Abschnitte 19-22 stehen in hier nicht gezeigter Weise mit Betätigungseinrichtungen wie oben beschrieben in Verbindung.

Die proximalen Abschnitte 19-22 sind nun so bewegbar, wie dies insbesondere in Fig. 2 gezeigt ist. Es können also die zwei auf der Oberseite des Hakens 10 liegenden proximalen Abschnitte 19 und 22 gegenüber den unten liegenden proximalen 20 und 21 entlang ihrer Längsachsen verschoben werden, so daß die distalen Abschnitte 13 und 14 bzw. 11 und 12 zwischeneinander einen Spalt bilden, so daß eine (Spreiz- und Klemm-) Zangenfunktion entsteht. Weiterhin können die links und rechts (in Fig. 1) liegenden proximalen Abschnitte 19 und 20 bzw. 21 und 22 so auseinander geklappt werden, daß zwischen den distalen Abschnitten 15 und 18 bzw. 16 und 17 Spalte freigegeben werden und diese distalen Abschnitte bzw. Zangenelemente zum Ergreifen von Gewebe oder auch zum Auseinanderspreizen von Gewebe verwendbar sind, wie dies insbesondere in Fig. 3 gezeigt ist. Man kann also mit dem hier gezeigten Instrument stumpfe Präparationen in einer Vielzahl von Bewegungsmöglichkeiten vornehmen, insbesondere Gewebe auf dem Haken 10 aufnehmen, es in zwei zueinander senkrechten Richtungen (gegebenenfalls auch gleichzeitig) aufspreizen oder in eben diesen beiden Richtungen zangenartig ergreifen.

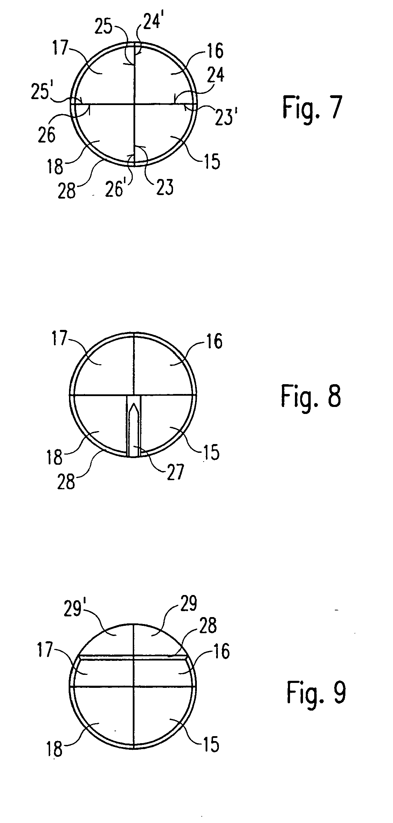

Vorzugsweise sind nun die distalen Abschnitte 15-18 - wie insbesondere in Fig. 7 gezeigt - ringsum mit einer Isolierung 28 ausgebildet und ansonsten aus Metall aufgebaut, so daß die bei geschlossenem Haken 10 innen liegenden Flächen Elektrodenabschnitte 23, 23'-26, 26' bilden. Diese Elektrodenabschnitte 23, 23'-26, 26' sind nun mit Hochfrequenzgeneratoren derart verbindbar, daß zwischen jeweils zwei aneinandergrenzenden Elektroden 23', 24; 24', 25; 25', 26 und 26`, 23 ein Koagulationsstrom dem Gewebe zuführbar ist, welches zwischen diesen beiden Elektrodenabschnitten bzw. distalen Abschnitten oder Zangenelementen eingeklemmt ist. Wenn man beispielsweise ein Gefäß zwischen zwei Zangenelementen 11-14 einklemmt und einen Koagulationsstrom zuführt, so kann das Gefäß an zwei Stellen gleichzeitig zur prophylaktischen Hämostase koaguliert werden. Nach einer solchen Koagulation kann dann eine Schneidelektrode 27, wie sie in den Figuren 4 bis 6 gezeigt ist, zwischen den auseinandergeklappten Zangenelementen 11, 12; 13, 14 hindurch in Richtung auf das eingeklemmte Gewebe geführt werden und ein entsprechender HF-Schneidstrom kann dann entweder monopolar (gegenüber einer Neutralelektrode) oder auch bipolar (gegenüber mindestens einem der Zangenelemente) zugeführt werden, um das eingeklemmte Gewebe zu durchtrennen. Nachdem eine Koagulation schon vorher stattgefunden hat, kann es dadurch nicht zu Blutungen kommen. Bei der in den Figuren 4 und 5 gezeigten Anordnung ist die Schneidelektrode 27 in einem Hohlraum zwischen den distalen Abschnitten 15-18 und den proximalen Abschnitten 19-22 angeordnet, wird also nur dann freigegeben, wenn die Zangenelemente 11-14 auseinandergefahren werden. In geschlossenem Zustand der Zange 10 ist die Elektode 27 somit geschützt.

Bei der in Fig. 8 gezeigten Anordnung ist die Schneidelektrode 27 messerartig ausgebildet und zwischen zwei der distalen Abschnitte 15 und 18 aufgenommen. Die Funktionsweise ist ansonsten entsprechend der nach Fig. 5, es ist also die Schneideinrichtung 27 in zusammengeklapptem Zustand des Hakens 10 geschützt.

Die Zangenelemente 11-14 können zusätzlich zu den dargestellten innenliegenden Elektrodenabschnitten 23, 23'-26, 26' auch nach außen am Gewebe anlegbare Elektrodenabschnitte 29, 29' aufweisen, die dazu benützt werden, Gewebe großflächig zu koagulieren, wenn dies notwendig sein sollte. Die Funktion der Zangenelemente 11-14 bzw. von deren Elektrodenabschnitten 23, 23'-26, 26' wird dadurch nicht beeinträchtigt, wenn zwischen der Außenelektrode 29 und den dazu gehörigen Elektrodenabschnitten eine Isolierung 28 vorgesehen ist. Selbstverständlich sind hier auch andere Kombinationen denkbar, so daß nicht nur mit einer Vorderseite des Hakens 10 sondern auch mit Seitenflächen oder auch nur mit seiner Spitze eine derartige Koagulation von Gewebe vorgenommen werden kann.

Der Koagulationsstrom kann auch in anderen Kombinationen der Elektrodeneinrichung zugeführt werden. Zum Beispiel kann man Gewebe zwischen zwei Zangenelementen 11 und 12 sowie 13 und 14 an zwei Stellen ergreifen und zwischen diesen Zangenpaaren einen Koagulationsstrom fließen lassen, um das gesamte zwischen diesen Zangenpaaren aufgenommene Gewebe zu koagulieren. Gerade in diesem Punkt, also im Hinblick auf Variabilität der Bestromung stellt die vorliegende Erfindung etwas besonderes dar.

### Bezugszeichenliste

- 10: Haken
- 11-14: Zangenelement
- 15-18: distaler Abschnitt
- 19-22: proximaler Abschnitt
- 23, 23'-26, 26': Elektrodenabschnitt
- 27: Schneidrichtung
- 28: Isolierung
- 29: Außenelektrode

## Patentansprüche

1. Chirurgisches Instrument, umfassend
Zangenelemente (11, 14) mit hakenförmigen distalen Abschnitten (15 - 18) mit freien Enden zum stumpfen Präparieren,
den distalen Abschnitten (15-18) zugeordnete proximale Abschnitte (19 - 22), die zum Bewegen der distalen Abschnitte (15 - 18) mit Betätigungseinrichtungen verbunden sind,
**dadurch gekennzeichnet, dass**
mindestens drei, insbesondere vier Zangenelemente (11, 14) mit hakenförmigen distalen Abschnitten (15-18) vorgesehen sind, die zum stumpfen Präparieren zu einem einzigen Haken (10) zusammenführbar und jeweils in Zweiergruppen als gegeneinander bewegbare Zangenelemente (11 - 14) zum Eingreifen oder Aufspreizen von Gewebe verwendbar sind.

2. Instrument nach Anspruch 1,
**dadurch gekennzeichnet, daß**
an den distalen Abschnitten (15-18) Elektrodeneinrichtungen (23-26) vorgesehen oder die distalen Abschnitten (15-18) als Elektrodeneinrichtungen ausgebildet sind, welche mit steuerbaren HF-Generatoren zur Zuführung eines Koagulationsstromes zu Gewebeabschnitten verbunden sind, die insbesondere von den Zangenelementen (11-14) erfaßt sind oder an die Zangenelemente (11-14) angelegt werden.

3. Instrument nach Anpruch 2,
**dadurch gekennzeichnet, daß**
die proximalen Abschnitte (19-22) und/oder die distalen Abschnitte (15-18) im wesentlichen ringsum isoliert sind.

4. Instrument nach einem der vorhergehenden Ansprüche,
**gekennzeichnet durch**
eine Schneideinrichtung (27), die gegenüber den Zangenelementen (11-14) zum Schneiden von Gewebe bewegbar ausgebildet ist.

5. Instrument nach Anspruch 4,
**dadurch gekennzeichnet, daß**
die Schneideeinrichtung (27) eine Elektrode umfaßt oder als Elektrode ausgebildet ist und mit einem steuerbaren HF-Generator zum Durchtrennen oder Schneiden von Gewebe mittels eines hochfrequenten elektrischen Stroms verbunden ist.

6. Instrument nach Anspruch 4,
**dadurch gekennzeichnet, daß**
die Schneideinrichtung (27) zwischen den distalen Abschnitten (15-18) und/oder den proximalen Abschnitten (19-22) aufnehmbar ist, wenn diese zu einem Haken (10) zusammengeführt sind.

7. Instrument nach einem der vorhergehenden Ansprüche,
**dadurch gekennzeichnet, daß**
die Betatigungseinrichtungen der proximalen Abschnitte (19-22) einen rohrförmigen Abschnitt umfassen, in welchem die proximalen Abschnitte (19-22) geführt und gehalten sind.

8. Instrument nach Anpruch 7,
**dadurch gekennzeichnet, daß**
ein einem proximalen Ende des rohrförmigen Abschnittes Manipulatoreinrichtungen zum Bewegen der distalen Abschnitte (15-18) vorgesehen sind.

## Claims

1. A surgical instrument comprising
forceps elements (11,14) with hooked distal portions (15-18) with free ends for blunt dissection,
proximal portions (19-22) which are associated with the distal portions (15-18) and which are connected to actuating means for moving the distal portions (15-18),
**characterised in that**
at least three, in particular four forceps elements (11,14) are provided with hooked distal portions (15-18) which, for blunt dissection, can be brought together to form a single hook (10) and can each be used in two groups as forceps elements (11-14) which can be moved relative to one another to grip or spread apart tissue.

2. An instrument according to Claim 1,
**characterised in that**
electrode devices (23-26) are provided on the distal portions (15-18) or the distal portions (15-18) are in the form of electrode devices which are connected with controllable HF generators for feeding a coagulation current to tissue sections which, in particular, are gripped by the forceps elements (11-14) or are applied on the forceps elements (11,14).

3. An instrument according to Claim 2,
**characterised in that**
the proximal portions (19-22) and/or the distal portions (15-18) are insulated substantially all round.

4. An instrument according to any one of the preceding Claims, **characterised by**
a cutting device (27) which for cutting tissue is designed to be movable relative to the forceps elements (11-14).

5. An instrument according to Claim 4,
**characterised in that**
the cutting device (27) comprises an electrode or is in the form of an electrode and is connected to a controllable HF generator for severing or cutting tissue by means of a highfrequency electric current.

6. An instrument according to Claim 4,
**characterised in that**
the cutting device (27) can be mounted between the distal portions (15-18) and/or the proximal portions (19-22) when the latter are brought together to form a hook (10).

7. An instrument according to any one of the preceding Claims, **characterised in that**
the actuating means of the proximal portions (19-22) comprises a tubular portion in which the proximal portions (19-22) are guided and retained.

8. An instrument according to Claim 7,
**characterised in that**
manipulator means are provided at [sic] a proximal end of the tubular portion for moving the distal portions (15-18).

## Revendications

1. Instrument chirurgical comprenant
des éléments de pince (11, 14) à parties distales en crochet (15 à 18) présentant des extrémités libres conçues pour la dissection mousse,
et, associées aux parties distales (15 à 18), des parties proximales (19 à 22) qui sont reliées à des dispositifs d'actionnement pour déplacer lesdites parties distales (15 à 18),
**caractérisé en ce que**
sont prévues au moins trois, en particulier quatre éléments de pince (11, 14) à parties distales en crochet (15 à 18), aptes à être rapprochées pour former un crochet unique (10) pour la dissection mousse et aptes à être utilisées par paires en tant qu'éléments de pince (11 à 14) mobiles relativement l'un à l'autre pour saisir ou écarter des tissus.

2. Instrument selon la revendication 1, **caractérisé en ce que** les parties distales (15 à 18) présentent des dispositifs à électrodes (23 à 26) ou **en ce que** les parties distales (15 à 18) se présentent sous la forme de dispositifs à électrodes reliés à des générateurs HF commandables pour délivrer un courant de coagulation aux parties de tissu qui sont saisies par les éléments de pince (11 à 14) ou contre lesquelles sont placés lesdits éléments de pince (11 à 14).

3. Instrument selon la revendication 2, **caractérisé en ce que** les parties proximales (19 à 22) et/ou les parties distales (15 à 18) sont isolées sur sensiblement toute leur périphérie.

4. Instrument selon l'une quelconque des revendications précédentes, **caractérisé par** un dispositif de coupe (27) conçu mobile par rapport aux éléments de pince (11 à 14) et destiné à couper les tissus.

5. Instrument selon la revendication 4, **caractérisé en ce que** le dispositif de coupe (27) comprend une électrode ou est conçu sous la forme d'une électrode et est relié à un générateur HF commandable pour couper des tissus au moyen d'un courant électrique haute fréquence.

6. Instrument selon la revendication 4, **caractérisé en ce que** le dispositif de coupe (27) peut être logé entre les parties distales (15 à 18) et/ou les parties proximales (19 à 22) lorsque celles-ci sont réunies en un crochet (10).

7. Instrument selon l'une quelconque des revendications précédentes, **caractérisé en ce que** les dispositifs d'actionnement des parties proximales (19 à 22) comprennent une partie tubulaire, dans laquelle les parties proximales (19 à 22) sont guidées et maintenues.

8. Instrument selon la revendication 7, **caractérisé en ce qu'**à une extrémité proximale de la partie tubulaire sont prévus des dispositifs de manipulation pour déplacer les parties distales (15 à 18).
